# EUROPEAN PATENT SPECIFICATION

(11) **EP 3 202 763 B1**
(45) Date of publication and mention of the grant of the patent: **13.12.2017**
(21) Application number: 15847030.2
(22) Date of filing: 28.09.2015
(51) Int. Cl.: C07D 285/08

(54) **METHOD FOR PRODUCING 5-AMINO-3 SUBSTITUTED-1,2,4-THIADIAZOLE**
VERFAHREN ZUR HERSTELLUNG VON 5-AMINO-3-SUBSTITUIERTEM 1,2,4-THIADIAZOL
PROCÉDÉ DE PRODUCTION DE 1,2,4-THIADIAZOLE À SUBSTITUTION 5-AMINO -3

(30) Priority: 02.10.2014 JP 2014204007
(43) Date of publication of application: 09.08.2017
(73) Proprietor: Fujifilm Corporation, Minato-ku Tokyo 106-8620 (JP)
(72) Inventor: HAGIO, Hiroyuki, Ashigarakami-gun Kanagawa 258-8577 (JP); SUGIMURA, Takanori, Ashigarakami-gun Kanagawa 258-8577 (JP); OMORI, Koji, Ashigarakami-gun Kanagawa 258-8577 (JP); MASAKI, Tomohito, Ashigarakami-gun Kanagawa 258-8577 (JP); MORI, Hideto, Ashigarakami-gun Kanagawa 258-8577 (JP)
(74) Representative: Hoffmann Eitle
(86) International application number: PCT/JP2015/077387
(87) International publication number: WO 2016/052439

(56) References cited:
- EP-A1- 0 064 582
- JP-A- H0 477 477
- JP-A- 2008 266 545
- JP-A- 2009 013 072
- JP-B2- H0 240 677
- KANAI, T. ET AL. BULLETIN OF THE CHEMICAL SOCIETY OF JAPAN vol. 66, no. 8, 1993, pages 2335 - 2338, XP002408735

## Description

### BACKGROUND OF THE INVENTION

### 1. Field of the Invention

The present invention relates to a method for manufacturing 5-amino-3 substituted-1,2,4-thiadiazole.

### 2. Description of the Related Art

5-Amino-3 substituted-1,2,4-thiadiazole is known as a starting material or a synthesis intermediate at the time of synthesizing a compound containing a 1,2,4-thiadiazole ring (for example, an azo compound).

For example, JP2008-266545A discloses, as an azo colorant having excellent color and fastness and showing a high molecular absorption coefficient, a 1,2,4-thiadiazole ring-containing azo colorant having a specific structure. JP2008-266545A also discloses that 5-amino-3-methylthio-1,2,4-thiadiazole is synthesized as a synthesis intermediate at the time of synthesizing the aforementioned azo colorant. Specifically, paragraph "0053" of JP2008-266545A discloses that, by simultaneously adding triethylamine and bromine dropwise to a mixture containing methanol, sodium thiocyanate, and a sulfuric acid solution of a specific amidine compound, 5-amino-3-methylthio-1,2,4-thiadiazole is synthesized. However, JP2008-266545A does not specifically disclose the pH conditions at the time of simultaneously adding triethylamine and bromine dropwise.

JP1990-40677B (JP-H02-40677B) discloses 7-substituted-3-cephem-4-carboxylic acid or a salt thereof which exhibits excellent antibacterial characteristics with respect to gram negative bacteria and a wide variety of pathogenic microorganisms including gram negative bacteria, and also discloses that, as a synthesis intermediate at the time of synthesizing 7-substituted-3-cephem-4-carboxylic acid or a salt thereof, 5-amino-3-methyl carboxylate-1,2,4-thiadiazole is synthesized. Specifically, "Manufacturing Example 8 (5)" in JP1990-40677B (JP-H02-40677B) discloses that, by adding bromine first to a mixture containing 1-methoxycarbonylformamidine hydrochloride, adding triethylamine thereto, and then adding a methanol solution of potassium thiocyanate thereto, 5-amino-3-methyl carboxylate-1,2,4-thiadiazole is synthesized.

### SUMMARY OF THE INVENTION

For the synthesis methods described in JP2008-266545A and JP1990-40677B (JP-H02-40677B), the further improvement of the yield and reaction reproducibility of a target substance is required.

An object of the present disclosure is to provide a method for manufacturing 5-amino-3 substituted-1,2,4-thiadiazole having excellent reaction reproducibility that makes it possible to manufacture 5-amino-3 substituted-1,2,4-thiadiazole in a high yield.

Specific means for achieving the above object is as follows.
<1> A method for manufacturing 5-amino-3 substituted-1,2,4-thiadiazole, comprising:
   obtaining a compound represented by the following Formula (II) by adding a brominating agent and a base in this order to a mixture comprising an alcohol, at least one selected from the group consisting of an amidine compound represented by the following Formula (I) and a mineral acid salt thereof, and an alkali metal salt of thiocyanic acid: 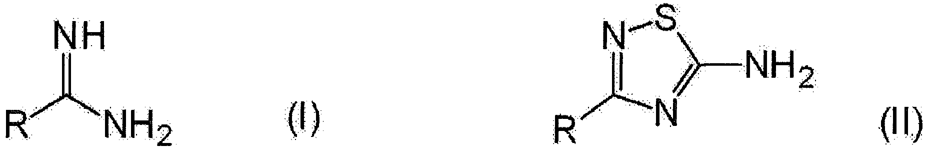 wherein, in Formulae (I) and (II), R represents an alkyl group having 1 to 5 carbon atoms or an alkylthio group having 1 to 5 carbon atoms.
<2> The method for manufacturing 5-amino-3 substituted-1,2,4-thiadiazole described in <1>, in which the mixture has a pH of from 9.0 to 11.5.
<3> The method for manufacturing 5-amino-3 substituted-1,2,4-thiadiazole described in <1> or <2>, in which the alcohol comprises a primary alcohol.
<4> The method for manufacturing 5-amino-3 substituted-1,2,4-thiadiazole described in any one of <1> to <3>, in which the brominating agent comprises bromine.
<5> The method for manufacturing 5-amino-3 substituted-1,2,4-thiadiazole described in any one of <1> to <4>, in which the base comprises a tertiary amine.
<6> The method for manufacturing 5-amino-3 substituted-1,2,4-thiadiazole described in any one of claims <1> to <5>, in which R in Formulae (I) and (II) represents a methyl group or a methylthio group.
<7> The method for manufacturing 5-amino-3 substituted-1,2,4-thiadiazole described in any one of <1> to <6>, in which the alcohol comprises a primary alcohol having 1 to 3 carbon atoms.
<8> The method for manufacturing 5-amino-3 substituted-1,2,4-thiadiazole described in any one of <1> to <7>, in which the base comprises a tertiary amine having 3 to 9 carbon atoms.
<9> The method for manufacturing 5-amino-3 substituted-1,2,4-thiadiazole described in any one of <1> to <8>, in which the alcohol comprises methanol.
<10> The method for manufacturing 5-amino-3 substituted-1,2,4-thiadiazole described in any one of <1> to <9>, in which the base comprises triethylamine.
<11> The method for manufacturing 5-amino-3 substituted-1,2,4-thiadiazole described in any one of <1> to <10>, in which the obtaining of the compound represented by Formula (II) comprises:
   adding the brominating agent and the base in this order to the mixture to obtain a reaction solution comprising the compound represented by Formula (II);
   concentrating the reaction solution; and
   adding water to the concentrated reaction solution to precipitate the compound represented by Formula (II).
<12> The method for manufacturing 5-amino-3 substituted-1,2,4-thiadiazole described in any one of <1> to <11>, in which an amount of the brominating agent added to the mixture is from 0.5-fold to 5.0-fold in terms of mole with respect to a total amount of the amidine compound represented by Formula (I) and the mineral acid salt thereof.
<13> The method for manufacturing 5-amino-3 substituted-1,2,4-thiadiazole described in any one of <1> to <12>, in which an amount of the base added to the mixture is from 0.5-fold to 6.0-fold in terms of mole with respect to a total amount of the amidine compound represented by Formula (I) and the mineral acid salt thereof.
<14> The method for manufacturing 5-amino-3 substituted-1,2,4-thiadiazole described in any one of <1> to <13>, in which in the mixture, a content of the alkali metal salt of thiocyanic acid is from 0.7-fold to 5.0-fold in terms of mole with respect to a total content of the amidine compound represented by Formula (I) and the mineral acid salt thereof.
<15> The method for manufacturing 5-amino-3 substituted-1,2,4-thiadiazole described in any one of <1> to <14>, in which in the mixture, a total content of the amidine compound represented by Formula (I), the mineral acid salt thereof, and the alkali metal salt of thiocyanic acid is from 1% by mass to 40% by mass with respect to a total amount of the mixture.

According to an aspect of the present invention, there is provided a method for manufacturing 5-amino-3 substituted-1,2,4-thiadiazole having excellent reaction reproducibility that makes it possible to manufacture 5-amino-3 substituted-1,2,4-thiadiazole in a high yield.

### DESCRIPTION OF THE PREFERRED EMBODIMENTS

Hereinafter, the method for manufacturing 5-amino-3 substituted-1,2,4-thiadiazole of the present disclosure will be specifically described.

In the present specification, a range of numerical values represented using "to" means a range which includes the numerical values listed before and after "to" as a lower limit and an upper limit.

The method for manufacturing 5-amino-3 substituted-1,2,4-thiadiazole of the present disclosure (hereinafter, referred to as a "manufacturing method of the present disclosure" as well) has a process of obtaining a compound represented by the following Formula (II) by adding a brominating agent and a base in this order to a mixture which contains an alcohol, at least either an amidine compound represented by Formula (I) or a mineral acid salt thereof, and an alkali metal salt of thiocyanic acid. If necessary, the manufacturing method of the present disclosure may have other processes.

The compound represented by the following Formula (II) is 5-amino-3 substituted-1,2,4-thiadiazole.

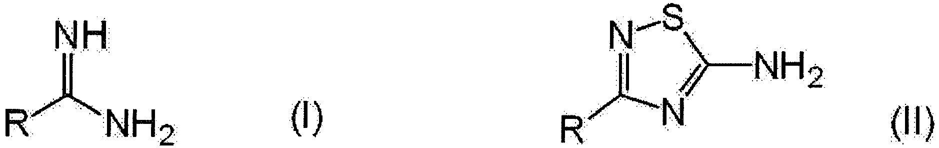

In Formulae (I) and (II), R represents an alkyl group having 1 to 5 carbon atoms or an alkylthio group having 1 to 5 carbon atoms.

In the present specification, at least either the amidine compound represented by Formula (I) or the mineral acid salt thereof is collectively called a "specific amidine" in some cases.

According to the manufacturing method of the present disclosure, the compound represented by Formula (II) that is a target substance can be manufactured in a high yield. Furthermore, the manufacturing method of the present disclosure is a manufacturing method excellent in reaction reproducibility.

Herein, "excellent in reaction reproducibility" means that, in a case where the manufacturing (synthesis) of the target substance is performed plural times, the target substance is stably manufactured (synthesized) practically in the same yield.

The above effect is assumed to be obtained for the following reason, but the present invention is not limited to the following assumption.

That is, in order to effectively carry on a reaction generating the compound represented by Formula (II) by using the specific amidine and the alkali metal salt of thiocyanic acid as starting materials and adding a brominating agent and a base to the mixture containing the starting materials, it is preferable to stably generate an amidine-Br complex which is an active intermediate.

By performing In-situ IR spectroscopy (onsite infrared spectroscopy), the inventors of the present invention found that the stability of the amidine-Br complex is greatly affected by the pH of a liquid generated by the amidine-Br complex.

From the viewpoint described above, in the manufacturing method of the present disclosure, to the mixture containing an alcohol, the specific amidine, and the alkali metal salt of thiocyanic acid, the brominating agent and the base are not simultaneously added, and instead, the brominating agent is added first. It is considered that, in this case, the pH of the liquid generated by the amidine-Br complex is further stabilized than in a case where the brominating agent and the base are simultaneously added, and the amidine-Br complex is stably generated. Then, to the liquid generated by the amidine-Br complex, the base is added. It is considered that, as a result, the amidine-Br complex and the alkali metal salt of thiocyanic acid effectively react with each other under basic conditions, and the compound (target substance) represented by Formula (II) is generated in a high yield.

It is considered that, for the aforementioned reason, in the manufacturing method of the present disclosure, the target substance is obtained in a high yield. Furthermore, it is considered that, in the manufacturing method of the present disclosure, the amidine-Br complex is stably generated, and hence the reaction reproducibility is maintained at a high level.

In the method of simultaneously adding the brominating agent and the base (for example, the method described in the aforementioned JP2008-266545A) to the mixture containing an alcohol, the specific amidine, and the alkali metal salt of thiocyanic acid, the yield and the reaction reproducibility of the target substance is further reduced than in the manufacturing method of the present disclosure. As the reason, it is considered that the simultaneous addition of the brominating agent and the base to the mixture changes the pH of the liquid that is supposed to be generated by the amidine-Br complex, and accordingly, the amidine-Br complex cannot be stably generated.

In addition, in a case where the brominating agent and the base are simultaneously added to the mixture, sometimes the pH of the liquid that is supposed to be generated by the amidine-Br complex becomes too high. It is considered that, accordingly, the reaction by which HBr is generated from the brominating agent takes precedence over the reaction by which the amidine-Br complex is generated from the brominating agent, and hence the efficiency of the generation of the amidine-Br complex is reduced.

In the method (for example, the method described in the aforementioned JP1990-40677B (JP-H02-40677B)) of adding the brominating agent and the base in this order not to a mixture which contains an alcohol, the specific amidine, and the alkali metal salt of thiocyanic acid but to a mixture which contains an alcohol and the specific amidine but does not contain the alkali metal salt of thiocyanic acid, and then adding the alkali metal salt of thiocyanic acid thereto, the yield and the reaction reproducibility of the target substance is further reduced than in the manufacturing method of the present disclosure. The reason is unclear but is considered to be as below. Because the amidine-Br complex has a short life, in the method of adding the alkali metal salt of thiocyanic acid later, the generation efficiency of the compound represented by Formula (II) is reduced, and hence the yield and the reaction reproducibility of the target substance is reduced.

In contrast, in the manufacturing method of the present disclosure, the alkali metal salt of thiocyanic acid pre-exists in the system. It is considered that, therefore, the amidine-Br complex having a short life and the pre-existing alkali metal salt of thiocyanic acid stably react with each other, and as a result, the compound represented by Formula (II) is stably generated.

In the manufacturing method of the present disclosure, a pH of the mixture is preferably 9.0 to 11.5.

If the pH of the mixture is equal to or higher than 9.0, the amidine-Br complex can more stably exist, and hence the yield and the reaction reproducibility can be further improved.

If the pH of the mixture is equal to or lower than 11.5, the generation of HBr from the brominating agent is inhibited, and the generation efficiency of the amidine-Br complex is further improved. Accordingly, if the pH of the mixture is equal to or lower than 11.5, the yield and the reaction reproducibility can be further improved.

The pH of the mixture is more preferably 9.5 to 11.0, and particularly preferably 10.0 to 11.0.

Herein, the pH of the mixture refers to the pH of the mixture whose temperature is controlled at 25°C.

Furthermore, the pH of the mixture refers to the pH established before the addition of the brominating agent and the base.

In the manufacturing method of the present disclosure, for example, in a case where the pH of the mixture obtained immediately after mixing an alcohol, the specific amidine, and the alkali metal salt of thiocyanic acid together is outside a range of 9.0 to 11.5, it is preferable to adjust the pH of the mixture within a range of 9.0 to 11.5 by adding an acid or a base to the mixture and to add the brominating agent and a base in this order to the mixture whose pH has been adjusted within a range of 9.0 to 11.5.

Examples of the acid used for the pH adjustment include hydrochloric acid, sulfuric acid, phosphoric acid, acetic acid, sulfonic acid, and the like.

Examples of the base used for the pH adjustment include the same bases as those, which will be described later, added after the brominating agent.

### <Compound represented by Formula (II)>

First, the compound represented by Formula (II) (5-amino-3-substituted-1,2,4-thiadiazole) which is a target substance of the manufacturing method of the present disclosure will be described.

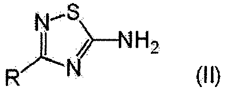

In Formula (II), R represents an alkyl group having 1 to 5 carbon atoms or an alkylthio group having 1 to 5 carbon atoms.

In Formula (II), examples of the alkyl group having 1 to 5 carbon atoms that is represented by R include a methyl group, an ethyl group, a propyl group (specifically, a n-propyl group or an i-propyl group), a butyl group, (specifically, a n-butyl group, an i-butyl group, a s-butyl group, or a t-butyl group), and a pentyl group.

From the viewpoint of the yield and the reaction reproducibility, the alkyl group having 1 to 5 carbon atoms is preferably an alkyl group having 1 to 3 carbon atoms, more preferably a methyl group or an ethyl group, and particularly preferably a methyl group.

In Formula (II), examples of the alkylthio group having 1 to 5 carbon atoms that is represented by R include a methylthio group, an ethylthio group, a propylthio group (specifically, a n-propylthio group or an i-propylthio group), a butylthio group (specifically, a n-butylthio group, an i-butylthio group, a s-butylthio group, or a t-butylthio group), and a pentylthio group.

From the viewpoint of the yield and the reaction reproducibility, the alkylthio group having 1 to 5 carbon atoms is preferably an alkylthio group having 1 to 3 carbon atoms, more preferably a methylthio group or an ethylthio group, and particularly preferably a methylthio group.

From the viewpoint of the yield and the reaction reproducibility, R in Formula (II) is preferably an alkyl group having 1 to 3 carbon atoms or an alkylthio group having 1 to 3 carbon atoms, more preferably a methyl group, an ethyl group, a methylthio group, or an ethylthio group, and particularly preferably a methyl group or a methylthio group.

The compound represented by Formula (II) is useful as a synthesis intermediate or a starting material of all compounds (for example, azo compounds) having a 1,2,4-thiadiazole ring.

### <Mixture>

Next, the mixture as an object to which the brominating agent and the base are added in the manufacturing method of the present disclosure will be described.

The mixture contains an alcohol, a specific amidine, and an alkali metal salt of thiocyanic acid.

The alcohol in the mixture is a component that functions as a reaction solvent.

The mixture may contain only one kind of alcohol or two or more kinds thereof.

From the viewpoint of the solubility of each component in the mixture, the alcohol is preferably a primary alcohol (methanol, ethanol, 1-propanol, 1-butanol, 1-pentanol, 1-hexanol, or the like), more preferably a primary alcohol having 1 to 3 carbon atoms, and particularly preferably methanol.

The content of the alcohol in the mixture is, with respect to the total amount of the mixture, preferably 40% to 95% by mass, more preferably 50% to 90% by mass, and particularly preferably 60% to 80% by mass.

(At least either amidine compound represented by Formula (I) or mineral acid salt thereof)

The mixture contains at least either an amidine compound (specific amidine) represented by Formula (I) or a mineral acid salt thereof.

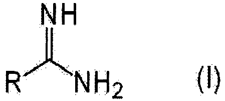

In Formula (I), R represents an alkyl group having 1 to 5 carbon atoms or an alkylthio group having 1 to 5 carbon atoms.

R in Formula (I) has the same definition as R in Formula (II), and a preferred range thereof is also the same.

Examples of the mineral acid salt of the amidine compound represented by Formula (I) include a sulfate of the amidine compound represented by Formula (I), a hydrochloride of the amidine compound represented by Formula (I), a phosphate of the amidine compound represented by Formula (I), and the like. Among these, a sulfate of the amidine compound represented by Formula (I) is particularly preferable.

The mixture may contain only one kind of the specific amidine or two or more kinds thereof.

The mixture may contain only either the amidine compound represented by Formula (I) or the mineral acid salt of the amidine compound represented by Formula (I) or contain both of them, as the specific amidine.

Next, the alkali metal salt of thiocyanic acid will be described.

Examples of the alkali metal salt of thiocyanic acid include sodium thiocyanate, potassium thiocyanate, and the like. Among these, sodium thiocyanate is particularly preferable.

The mixture may contain only one kind of the alkali metal salt of thiocyanic acid or two or more kinds thereof.

In the mixture, the content of the alkali metal salt of thiocyanic acid with respect to the total content of the amidine compound represented by Formula (I) and the mineral acid salt thereof is preferably 0.7-fold to 5.0-fold in terms of mole.

Hereinafter, a case where the content of the alkali metal salt of thiocyanic acid with respect to the total content of the amidine compound represented by Formula (I) and the mineral acid salt thereof is 0.7-fold to 5.0-fold in terms of mole is described as "a molar ratio [alkali metal salt of thiocyanic acid/specific amidine] is 0.7 to 5.0".

The molar ratio [alkali metal salt of thiocyanic acid/specific amidine] in the mixture is preferably 0.7 to 5.0, more preferably 1.0 to 4.0, even more preferably 1.5 to 3.5, and particularly preferably 2.0 to 3.0.

In the mixture, the total content of the specific amidine and the alkali metal salt of thiocyanic acid is, with respect to the total amount of the mixture, preferably 1% to 40% by mass, more preferably 5% to 35% by mass, and particularly preferably 10% to 30% by mass.

In the present specification, the total content of the specific amidine and the alkali metal salt of thiocyanic acid means the total content of the amidine compound represented by Formula (I), the mineral acid salt thereof, and the alkali metal salt of thiocyanic acid in the mixture.

More specifically, in a case where the mixture contains only either the amidine compound represented by Formula (I) or the mineral acid salt thereof, the total content of the specific amidine and the alkali metal salt of thiocyanic acid refers to the total content of only either the amidine compound or the mineral acid salt thereof and the alkali metal salt of thiocyanic acid. In a case where the mixture contains both of the amidine compound represented by Formula (I) and the mineral acid salt thereof, the total content of the specific amidine and the alkali metal salt of thiocyanic acid refers to the total content of both of the amidine compound and the mineral acid salt thereof and the alkali metal salt of thiocyanic acid.

The mixture may contain other components in addition to the alcohol, the specific amidine, and the alkali metal salt of thiocyanic acid.

Examples of other components described above include a solvent other than an alcohol, an acid or base used for adjusting the pH of the mixture, and the like. Specific examples of each of the acid and base are as described above.

Examples of the solvent other than an alcohol include ethyl acetate, tetrahydrofuran, acetonitrile, 2-butanone, and the like.

In the mixture, the content of the solvent other than an alcohol is preferably equal to or less than 20% by mass, more preferably equal to or less than 10% by mass, even more preferably equal to or less than 5% by mass, and particularly preferably equal to or less than 1% by mass.

### <Brominating agent>

In the manufacturing method of the present disclosure, a brominating agent is added first to the aforementioned mixture.

Examples of the brominating agent include bromine, bromodimethylsulfonium bromide, 1,3-dibromo-5,5-dimethylhydantoin, N-bromosuccinimide, 1-bromo-3-chloro-5,5-dimethylhyndantoin, and the like. Among these, bromine is preferable.

In view of the generation efficiency of the amidine-Br complex, the amount of the brominating agent added is, with respect to the amount of the specific amidine, preferably 0.5-fold to 5.0-fold in terms of mole, more preferably 1.0-fold to 4.0-fold in terms of mole, even more preferably 1.5-fold to 3.5-fold in terms of mole, and particularly preferably 2.0-fold to 3.0-fold in terms of mole.

In the present specification, the "amount of the specific amidine" means the total amount of the amidine compound represented by Formula (I) and the mineral acid salt thereof in the mixture. More specifically, in a case where the mixture contains only either the amidine compound represented by Formula (I) or the mineral acid salt thereof, the "amount of the specific amidine compound" refers to the amount of only either the amidine compound or the mineral acid salt thereof. In a case where the mixture contains both of the amidine compound represented by Formula (I) and the mineral acid salt thereof, the amount of the specific amidine refers to the total amount of both of the amidine compound and the mineral acid salt thereof.

In the manufacturing method of the present disclosure, from the viewpoint further improving the yield and the reaction reproducibility, it is preferable that the brominating agent is added dropwise to the mixture.

More specifically, the brominating agent is added dropwise to the mixture over 0.2 hours to 5.0 hours (more preferably 0.5 hours to 4.0 hours, even more preferably 1.0 hour to 3.0 hours, and particularly preferably 1.0 hour to 2.0 hours).

At the time of adding the brominating agent to the mixture, the temperature of the mixture and the brominating agent is preferably -5°C to 10°C, and more preferably 0°C to 10°C.

### <Base>

In the manufacturing method of the present disclosure, a base is added to the mixture to which the brominating agent has been added.

The base is not particularly limited, and examples thereof include a tertiary amine, an alkali metal alkoxide (for example, sodium methoxide or sodium ethoxide), and the like.

Among these, from the viewpoint of the yield and the reaction reproducibility, a tertiary amine is preferable.

Examples of the tertiary amine include trimethylamine, triethylamine, tripropylamine, triisopropylamine, tributylamine, dimethylethylamine, diethylmethylamine, dimethylpropylamine, methyldiisopropylamine, and the like.

Among these, from the viewpoint of the yield and the reaction reproducibility, a tertiary amine having 3 to 9 carbon atoms is preferable, a tertiary amine having 3 to 6 carbon atoms is even more preferable, and triethylamine is particularly preferable.

The amount of the base added is, with respect to the amount of the specific amidine, preferably 0.5-fold to 6.0-fold in terms of mole, more preferably 1.0-fold to 6.0-fold in terms of mole, even more preferably 2.0-fold to 5.5-fold in terms of mole, and particularly preferably 3.0-fold to 5.0-fold in terms of mole.

The meaning of the "amount of the specific amidine" is as described above.

In the manufacturing method of the present disclosure, from the viewpoint of further improving the yield and the reaction reproducibility, the base is preferably added dropwise to the mixture.

More preferably, the base is added dropwise to the mixture over 0.2 hours to 5.0 hours (more preferably 0.5 hours to 4.0 hours, even more preferably 1.0 hour to 3.0 hours, and particularly preferably 1.0 hour to 2.0 hours).

At the time of adding the base to the mixture to which the brominating agent has been added, the temperature of the mixture, the brominating agent, and the base is preferably -5°C to +10°C, and more preferably 0°C to +10°C.

In the manufacturing method of the present disclosure, as described above, by sequentially adding the brominating agent and the base to the mixture, a reaction of generating the compound represented by Formula (II) is performed.

The reaction time is preferably 0.2 to 5.0 hours, more preferably 0.5 hours to 4.0 hours, even more preferably 1.0 hour to 3.0 hours, and particularly preferably 1.5 hours to 2.5 hours.

The reaction temperature is preferably -5°C to +10°C, and more preferably 0°C to +10°C.

The reaction can be stopped by a known method such as a method of adding a mixture of sodium sulfite and water to the reaction solution.

In the manufacturing method of the present disclosure, the process of obtaining the compound represented by Formula (II) preferably has a step of obtaining a reaction solution containing the compound represented by Formula (II) by adding the brominating agent and the base in this order to the mixture and a step of precipitating the compound represented by Formula (II) by adding water to the reaction solution.

In this way, the compound (target substance) represented by Formula (II) can be more easily extracted from the reaction solution.

The step of precipitating the compound represented by Formula (II) is more preferably a step of precipitating the compound represented by Formula (II) by adding water to the reaction solution and cooling the reaction solution, to which water is added, to 0°C to 10°C, for example.

In the manufacturing method of the present disclosure, the process of obtaining the compound represented by Formula (II) preferably has a step of obtaining a reaction solution containing the compound represented by Formula (II) by adding the brominating agent and the base in this order to the mixture, a step of concentrating the reaction solution, and a step of precipitating the compound represented by Formula (II) by adding water to the concentrated reaction solution.

If the process of obtaining the compound represented by Formula (II) has the step of concentrating the reaction solution, the yield and the reaction reproducibility (particularly, the reaction reproducibility) are further improved.

In a case where the process of obtaining the compound represented by Formula (II) has the step of concentrating the reaction solution, the step of precipitating the compound represented by Formula (II) is also preferably a step of precipitating the compound represented by Formula (II) by adding water to the concentrated reaction solution and cooling the reaction solution, to which water is added, to 0°C to 10°C, for example.

The reaction solution can be concentrated by a general method in which a solvent (mainly the alcohol in the manufacturing method of the present disclosure) is distilled away under the condition with a reduced pressure.

When the solvent is distilled away, the temperature of the reaction solution is preferably 20°C to 80°C, more preferably 30°C to 70°C, and particularly preferably 40°C to 60°C.

The temperature of water added to the concentrated reaction solution is preferably 20°C to 80°C, more preferably 30°C to 70°C, and particularly preferably 40°C to 60°C. Examples

Hereinafter, the present invention will be more specifically described based on examples, but the present invention is not limited to the following examples as long as the present invention does not depart from the gist thereof.

In the following description, unless otherwise specified, "%" refers to "% by mass".

### [Example 1]

A brominating agent and a base were added dropwise in this order to a mixture containing an alcohol, a specific amidine, and an alkali metal salt of thiocyanic acid, thereby performing the synthesis of 5-amino-3-methylthio-1,2,4-thiadiazole. The synthesis was performed twice. The details thereof will be described below.

### <First synthesis>

13.9 g (0.05 mol) of sulfate of amidine A was added to 100 mL of methanol at room temperature, and 15.4 mL (0.11 mol) of triethylamine was added thereto. 10.5 g (0.13 mol) of sodium thiocyanate was added to the obtained solution at room temperature, thereby obtaining a mixture.

The amidine A is an amidine compound represented by Formula (I) in which R is a methylthio group. The sulfate of the amidine A is an example of the specific amidine.

Then, first, as a brominating agent, bromine (19.2 g, 0.12 mol) was added dropwise to the mixture at an internal temperature of 5°C. Thereafter, as a base, 26.4 mL (0.19 mol) of triethylamine was added dropwise thereto at the same internal temperature of 5°C.

The obtained solution was stirred for 2 hours at 5°C, and then sodium sulfite (1.9 g)/10 mL of water were added dropwise thereto such that the reaction was stopped. Subsequently, 500 mL of water was added thereto at an internal temperature of 5°C, and the solvent was distilled away under the condition with a reduced pressure (-90 kPa, internal temperature: 50°C). Thereafter, water was added dropwise thereto at an internal temperature of 50°C, and then the solution was cooled to 5°C and stirred for 30 minutes. As a result, a crystal was precipitated. The precipitated crystal was collected by filtration and washed twice with 50 mL of water, thereby obtaining 10.3 g (yield: 70%) of a whitish yellow crystal.

The obtained whitish yellow crystal was analyzed by high-performance liquid chromatography (HPLC) under the following conditions and NMR. As a result, it was confirmed that the obtained whitish yellow crystal is 5-amino-3-methylthio-1,2,4-thiadiazole (hereinafter, referred to as "MSTY" as well) which is the compound represented by Formula (II).

### (HPLC conditions)

### -Reaction tracking conditions-

- Column: TSKgel (registered trademark) ODS-80TM (column size: 4.6 mm I. D. × 25 cm) manufactured by Tosoh Corporation
- Flow rate: 1 mL/min
- Detection wavelength: 230 nm
- Column temperature: 40°C
- Solution A: ultrapure water/methanol = 9/1 (volume ratio, containing 0.1% by volume of acetic acid and 0.1% by volume of triethylamine)
- Solution B: ultrapure water/methanol = 1/9 (volume ratio, containing 0.1% by volume of acetic acid and 0.1% by volume of triethylamine)
- Gradient conditions: 0.1 min (solution A/solution B = 80/20 (volume ratio))→10.0 min (solution A/solution B = 80/20 (volume ratio))→40.0 min (solution A/solution B = 0/100 (volume ratio))→45 min (solution A/solution B = 0/100 (volume ratio))→45 min (stop)
- Retention time: amidine A; 2.94 min, NaSCN; 3.41 min, MSTY: 11.0 min

### -Crystal analysis conditions-

•Column: TSKgel (registered trademark) ODS-80TM (column size: 4.6 mm I. D. × 25 cm) manufactured by Tosoh Corporation
•Flow rate: 1 mL/min
•Detection wavelength: 254 nm
•Column temperature: 40°C
•Solution A: ultrapure water (containing 0.1% by volume of acetic acid and 0.1% by volume of triethylamine)
•Solution B: acetonitrile (containing 0.1% by volume of acetic acid and 0.1% by volume of triethylamine)
•Gradient conditions: 0.01 min (solution A/solution B = 80/20 (volume ratio))→65.0 min (solution A/solution B = 10/90 (volume ratio))→75.00 min (solution A/solution B = 0/100 (volume ratio))→75.01 min (solution A/solution B = 80/20 (volume ratio))→90.00 min (solution A/solution B = 80/20 (volume ratio))→90.01 min (stop)
•Retention time: MSTY; 5.14 min, unknown impurity; 15.72 min

### (NMR measurement result: 400 MHz, CDCl₃)

5.7 (brs, 2H), 2.6 (s, 3H)

### (Measurement of pH of mixture)

The pH (25°C) of the mixture to which the brominating agent (bromine) had not yet been added dropwise was measured using a pH meter. The results are shown in Table 1.

### (Confirmation of generation of amidine-Br complex)

In the whole process of sequentially adding bromine and triethylamine dropwise to the aforementioned mixture, an In situ IR verification experiment was performed under the following conditions. In this way, the generation of an amidine-Br complex as an active intermediate was confirmed and judged according to the following judgment standards. The results are shown in Table 1.

In the following judgment standards, a "maximum value" refers to a maximum value of the total area of peaks resulting from the amidine-Br complex, among the total area obtained in the first and second synthesises of Example 1, the total area obtained in the first and second synthesises of Example 2, and the total area obtained in the first and second synthesises of Example 3.

### -Conditions of In situ IR verification experiment-

- Device name: ReactIR™ iC10 (manufactured by Mettler-Toledo International Inc.)
- Probe: DiComp™ (6 mm, diamond, manufactured by Mettler-Toledo International Inc.)
- Measurement interval: 2 minutes

### -Judgment standards-

A: The total area of peaks resulting from the amidine-Br complex was equal to or greater than 70% and equal to or less than 100% with respect to the maximum value, and the generation of the amidine-Br complex as an active intermediate was confirmed.
B: The total area of peaks resulting from the amidine-Br complex was equal to or greater than 30% and less than 70% with respect to the maximum value, and the generation of the amidine-Br complex as an active intermediate was confirmed.
C: The total area of peaks resulting from the amidine-Br complex was equal to or greater than 0% and less than 30% with respect to the maximum value.

### <Second synthesis>

According to the same procedure as in the first synthesis, the second synthesis was performed.

The yield, the confirmation result of the generation of the amidine-Br complex, and the color of the product are shown in Table 1.

### <Evaluation of reaction reproducibility>

Based on the yield of the first synthesis and the yield of the second synthesis, the reaction reproducibility was evaluated according to the following evaluation standards.

### -Evaluation standards of reaction reproducibility-

A: A difference between the yield of the first synthesis and the yield of the second synthesis was equal to or less than 3%, and hence the reaction reproducibility was excellent.
B: A difference between the yield of the first synthesis and the yield of the second synthesis was greater than 3%, and hence the reaction reproducibility was poor.

### [Example 2]

The same operation as in Example 1 was performed except that, in Example 1, 15.4 mL (0.11 mol) of triethylamine added in the step of obtaining a mixture and 26.4 mL (0.19 mol) of triethylamine added dropwise to the mixture after bromine were changed to 21.2 mL (0.11 mol) of a methanol solution containing 28% sodium methoxide and 36.7 mL (0.19 mol) of a methanol solution containing 28% sodium methoxide respectively. The results are shown in Table 1.

### [Example 3]

The same operation as in Example 1 was performed except that, in Example 1, the sulfate of the amidine A was changed to a sulfate of amidine B in the same mole number such that a compound represented by Formula (II) in which R was a methyl group was synthesized. The results are shown in Table 1.

The amidine B is an amidine compound represented by Formula (I) in which R is a methyl group.

### [Comparative Example 1]

By simultaneously adding a brominating agent and a base dropwise to a mixture containing an alcohol, a specific amidine, and an alkali metal salt of thiocyanic acid, the synthesis of 5-amino-3-methylthio-1,2,4-thiadiazole was performed. The synthesis was performed twice. The details thereof will be described below.

### <First synthesis>

13.9 g (0.05 mol) of a sulfate of amidine A was added to 100 mL of methanol at room temperature, and 15.4 mL (0.11 mol) of triethylamine was added thereto. 10.5 g (0.13 mol) of sodium thiocyanate was added to the obtained solution at room temperature, thereby obtaining a mixture.

At an internal temperature of 5°C, 14 mL (0.1 mol) of triethylamine and 16.3 g (0.102 mol) of bromine were simultaneously added dropwise to the obtained mixture. The obtained solution was stirred for 2 hours at room temperature, thereby obtaining a reaction solution. The obtained reaction solution was added to 1,000 mL of cold water and stirred for 30 minutes, and as a result, a crystal was precipitated. The precipitated crystal was collected by filtration, sufficiently washed with water, and dried, thereby obtaining 5.1 g (yield: 35%) of a yellow crystal.

The obtained yellow crystal was analyzed in the same manner as in Example 1. As a result, it was confirmed that the obtained yellow crystal is 5-amino-3-methylthio-1,2,4-thiadiazole (MSTY).

In the synthesis process described above, the same measurement as in Example 1 was performed. The results are shown in Table 1.

### <Second synthesis>

According to the same procedure as in the first synthesis, the second synthesis was performed.

The yield, the confirmation result of the generation of the amidine-Br complex, and the color of the product are shown in Table 1.

### <Evaluation of reaction reproducibility>

Based on the yield of the first synthesis and the yield of the second synthesis, the reaction reproducibility was evaluated in the same manner as in Example 1. The results are shown in Table 1.

### [Comparative Example 2]

The same operation as in Comparative Example 1 was performed except that, in Comparative Example 1, the operation performed after triethylamine and bromine were simultaneously added dropwise was changed to the following operation. The results are shown in Table 1.

### -Operation performed after triethylamine and bromine are simultaneously added dropwise (Comparative Example 2)-

The solution obtained by simultaneously adding triethylamine and bromine dropwise was stirred for 2 hours at 5°C, and then sodium sulfite (1.9 g)/10 mL of water were added dropwise thereto such that the reaction was stopped. Then, 500 mL of water was added thereto at an internal temperature of 5°C, and the solvent was distilled away under the condition with a reduced pressure (-90 kPa, internal temperature: 50°C). Thereafter, water was added dropwise thereto at an internal temperature of 50°C, and then the solution was cooled to 5°C and stirred for 30 minutes. As a result, a crystal was precipitated. The precipitated crystal was collected by filtration and washed twice with 50 mL of water, thereby obtaining a yellow crystal.

### [Comparative Example 3]

By adding a brominating agent and a base dropwise in this order to a mixture containing an alcohol and a specific amidine, and then adding an alkali metal salt of thiocyanic acid thereto, the synthesis of 5-amino-3-methylthio-1,2,4-thiadiazole was performed. The synthesis was performed twice. The details will be described below.

### <First synthesis>

100 mL of methanol was cooled to 5°C, and 13.9 g (0.05 mol) of a sulfate of amidine A and 15.4 mL (0.11 mol) of triethylamine were added thereto at an internal temperature of 5°C, thereby obtaining a mixture. At an internal temperature of 5°C, 16.3 g (0.102 mol) of bromine and 14 mL (0.1 mol) of triethylamine were added dropwise in this order to the obtained solution.

To the obtained solution, 10.5 g (0.13 mol) of sodium thiocyanate was added at an internal temperature of 5°C.

The obtained solution was stirred for 2 hours at 5°C, and then sodium sulfite (1.9 g)/10 mL of water were added dropwise thereto such that the reaction was stopped. Then, 500 mL of water was added thereto at an internal temperature of 5°C, and the solvent was distilled away under the condition with a reduced pressure (-90 kPa, internal temperature: 50°C). Thereafter, water was added dropwise thereto at an internal temperature of 50°C, and then the solution was cooled to 5°C and stirred for 30 minutes. As a result, a crystal was precipitated. The precipitated crystal was collected by filtration and washed twice with 50 mL of water, thereby obtaining a yellow crystal.

The obtained yellow crystal was analyzed in the same manner as in Example 1. As a result, it was confirmed that the obtained yellow crystal is 5-amino-3-methylthio-1,2,4-thiadiazole (MSTY).

In the synthesis process described above, the same measurement as in Example 1 was performed. The results are shown in Table 1.

### <Second synthesis>

According to the same procedure as in the first synthesis, the second synthesis was performed.

The yield, the confirmation result of the generation of the amidine-Br complex, and the color of the product are shown in Table 1.

**[Table 1]**

| | R in Formula (I) | Base | Time to add alkali metal salt of thiocyanic acid | pH before dropwise addition of brominating agent | Order of dropwise addition of brominating agent and base | Method for extracting target substance | Generation of active intermediate (amidine-Br complex) | | Yield | | Reaction reproducibility | Color of product | |
|---|---|---|---|---|---|---|---|---|---|---|---|---|---|
| Example 1 | -SCH₃ | Triethylamine | Before dropwise addition of brominating agent and base (at the time of preparing mixture) | 11.0 | Brominating agent→base | Concentration and then addition of water | First synthesis | A | First synthesis | 70% | A | First synthesis | Whitish yellow |
| | | | | | | | Second synthesis | A | Second synthesis | 71% | | Second synthesis | Whitish yellow |
| Example 2 | -SCH₃ | Methanol solution of 28% sodium methoxide | Before dropwise addition of brominating agent and base (at the time of preparing mixture) | 11.0 | Brominating agent→base | Concentration and then addition of water | First synthesis | A | First synthesis | 50% | A | First synthesis | White |
| | | | | | | | Second synthesis | A | Second synthesis | 52% | | Second synthesis | White |
| Example 3 | -CH₃ | Triethylamine | Before dropwise addition of brominating agent and base (at the time of preparing mixture) | 11.0 | Brominating agent→base | Concentration and then addition of water | First synthesis | A | First synthesis | 70% | A | First synthesis | Whitish yellow |
| | | | | | | | Second synthesis | A | Second synthesis | 70% | | Second synthesis | Whitish yellow |
| Comparative Example 1 | -SCH₃ | Triethylamine | Before dropwise addition of brominating agent and base (at the time of preparing mixture) | 11.0 | Simultaneous | Addition of water without concentration | First synthesis | B | First synthesis | 35% | B | First synthesis | Yellow |
| | | | | | | | Second synthesis | B | Second synthesis | 20% | | Second synthesis | Yellow |
| Comparative Example 2 | -SCH₃ | Triethylamine | Before dropwise addition of brominating agent and base (at the time of preparing mixture) | 11.0 | Simultaneous | Concentration and then addition of water | First synthesis | B | First synthesis | 30% | B | First synthesis | Yellow |
| | | | | | | | Second synthesis | B | Second synthesis | 38% | | Second synthesis | Yellow |
| Comparative Example 3 | -SCH₃ | Triethylamine | After dropwise addition of brominating agent and base | 11.0 | Brominating agent→base | Concentration and then addition of water | First synthesis | B | First synthesis | 40% | B | First synthesis | Yellow |
| | | | | | | | Second synthesis | B | Second synthesis | 35% | | Second synthesis | Yellow |

As shown in Table 1, in Examples 1 to 3 in which a brominating agent and a base were added in this order to a mixture containing an alcohol, a specific amidine, and an alkali metal salt of thiocyanic acid, a product (target substance: 5-amino-3 substituted-1,2,4-thiadiazole) could be obtained in a high yield with excellent reaction reproducibility.

In contrast, in Comparative Examples 1 and 2 in which a brominating agent and a base were simultaneously added to a mixture containing an alcohol, a specific amidine, and an alkali metal salt of thiocyanic acid, the yield and the reaction reproducibility were reduced.

Furthermore, in Comparative Example 3 in which an alkali metal salt of thiocyanic acid was added after the addition of a brominating agent and a base, the yield and the reaction reproducibility were also reduced.

## Claims

1. A method for manufacturing 5-amino-3 substituted-1,2,4-thiadiazole, comprising:
obtaining a compound represented by the following Formula (II) by adding a brominating agent and a base in this order to a mixture comprising an alcohol, at least one selected from the group consisting of an amidine compound represented by the following Formula (I) and a mineral acid salt thereof, and an alkali metal salt of thiocyanic acid: 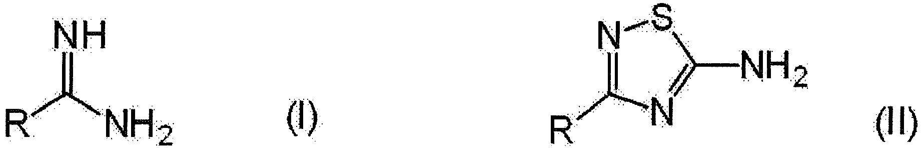
wherein, in Formulae (I) and (II), R represents an alkyl group having 1 to 5 carbon atoms or an alkylthio group having 1 to 5 carbon atoms.

2. The method for manufacturing 5-amino-3 substituted-1,2,4-thiadiazole according to claim 1, wherein the mixture has a pH of from 9.0 to 11.5.

3. The method for manufacturing 5-amino-3 substituted-1,2,4-thiadiazole according to claim 1 or 2, wherein the alcohol comprises a primary alcohol.

4. The method for manufacturing 5-amino-3 substituted-1,2,4-thiadiazole according to any one of claims 1 to 3, wherein the brominating agent comprises bromine.

5. The method for manufacturing 5-amino-3 substituted-1,2,4-thiadiazole according to any one of claims 1 to 4, wherein the base comprises a tertiary amine.

6. The method for manufacturing 5-amino-3 substituted-1,2,4-thiadiazole according to any one of claims 1 to 5, wherein R in Formulae (I) and (II) represents a methyl group or a methylthio group.

7. The method for manufacturing 5-amino-3 substituted-1,2,4-thiadiazole according to any one of claims 1 to 6, wherein the alcohol comprises a primary alcohol having 1 to 3 carbon atoms.

8. The method for manufacturing 5-amino-3 substituted-1,2,4-thiadiazole according to any one of claims 1 to 7, wherein the base comprises a tertiary amine having 3 to 9 carbon atoms.

9. The method for manufacturing 5-amino-3 substituted-1,2,4-thiadiazole according to any one of claims 1 to 8, wherein the alcohol comprises methanol.

10. The method for manufacturing 5-amino-3 substituted-1,2,4-thiadiazole according to any one of claims 1 to 9, wherein the base comprises triethylamine.

11. The method for manufacturing 5-amino-3 substituted-1,2,4-thiadiazole according to any one of claims 1 to 10, wherein the obtaining of the compound represented by Formula (II) comprises:
adding the brominating agent and the base in this order to the mixture to obtain a reaction solution comprising the compound represented by Formula (II);
concentrating the reaction solution; and
adding water to the concentrated reaction solution to precipitate the compound represented by Formula (II).

12. The method for manufacturing 5-amino-3 substituted-1,2,4-thiadiazole according to any one of claims 1 to 11, wherein an amount of the brominating agent added to the mixture is from 0.5-fold to 5.0-fold in terms of mole with respect to a total amount of the amidine compound represented by Formula (I) and the mineral acid salt thereof.

13. The method for manufacturing 5-amino-3 substituted-1,2,4-thiadiazole according to any one of claims 1 to 12, wherein an amount of the base added to the mixture is from 0.5-fold to 6.0-fold in terms of mole with respect to a total amount of the amidine compound represented by Formula (I) and the mineral acid salt thereof.

14. The method for manufacturing 5-amino-3 substituted-1,2,4-thiadiazole according to any one of claims 1 to 13, wherein in the mixture, a content of the alkali metal salt of thiocyanic acid is from 0.7-fold to 5.0-fold in terms of mole with respect to a total content of the amidine compound represented by Formula (I) and the mineral acid salt thereof.

15. The method for manufacturing 5-amino-3 substituted-1,2,4-thiadiazole according to any one of claims 1 to 14, wherein in the mixture, a total content of the amidine compound represented by Formula (I), the mineral acid salt thereof, and the alkali metal salt of thiocyanic acid is from 1% by mass to 40% by mass with respect to a total amount of the mixture.

## Patentansprüche

1. Verfahren zur Herstellung von 5-Amino-3 substituiertem 1,2,4-Thiadiazol, umfassend:
Erhalten einer Verbindung der nachstehenden Formel (II) durch Zugabe eines Bromierungsmittels und einer Base in dieser Reihenfolge zu einer Mischung, umfassend einen Alkohol, zumindest eines, ausgewählt aus der Gruppe bestehend aus einer Amidinverbindung der nachstehenden Formel (I) und eines Mineralsäuresalzes davon, und ein Alkalimetallsalz von Thiocyansäure: 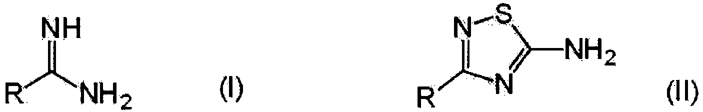
wobei in den Formeln (I) und (II) R eine Alkylgruppe mit 1 bis 5 Kohlenstoffatomen oder eine Alkylthiogruppe mit 1 bis 5 Kohlenstoffatomen darstellt.

2. Verfahren zur Herstellung von 5-Amino-3 substituiertem 1,2,4-Thiadiazol gemäss Anspruch 1, wobei die Mischung einen pH-Wert von 9,0 bis 11,5 aufweist.

3. Verfahren zur Herstellung von 5-Amino-3 substituiertem 1,2,4-Thiadiazol gemäss Anspruch 1 oder 2, wobei der Alkohol einen primären Alkohol umfasst.

4. Verfahren zur Herstellung von 5-Amino-3 substituiertem 1,2,4-Thiadiazol gemäss irgendeinem der Ansprüche 1 bis 3, wobei das Bromierungsmittel Brom umfasst.

5. Verfahren zur Herstellung von 5-Amino-3 substituiertem 1,2,4-Thiadiazol gemäss irgendeinem der Ansprüche 1 bis 4, wobei die Base ein tertiäres Amin umfasst.

6. Verfahren zur Herstellung von 5-Amino-3 substituiertem 1,2,4-Thiadiazol gemäss irgendeinem der Ansprüche 1 bis 5, wobei R in den Formeln (I) und (II) eine Methylgruppe oder eine Methylthiogruppe darstellt.

7. Verfahren zur Herstellung von 5-Amino-3 substituiertem 1,2,4-Thiadiazol gemäss irgendeinem der Ansprüche 1 bis 6, wobei der Alkohol einen primären Alkohol mit 1 bis 3 Kohlenstoffatomen umfasst.

8. Verfahren zur Herstellung von 5-Amino-3 substituiertem 1,2,4-Thiadiazol gemäss irgendeinem der Ansprüche 1 bis 7, wobei die Base ein tertiäres Amin mit 3 bis 9 Kohlenstoffatomen umfasst.

9. Verfahren zur Herstellung von 5-Amino-3 substituiertem 1,2,4-Thiadiazol gemäss irgendeinem der Ansprüche 1 bis 8, wobei der Alkohol Methanol umfasst.

10. Verfahren zur Herstellung von 5-Amino-3 substituiertem 1,2,4-Thiadiazol gemäss irgendeinem der Ansprüche 1 bis 9, wobei die Base Triethylamin umfasst.

11. Verfahren zur Herstellung von 5-Amino-3 substituiertem 1,2,4-Thiadiazol gemäss irgendeinem der Ansprüche 1 bis 10, wobei das Erhalten der Verbindung der Formel (II) umfasst:
die Zugabe des Bromierungsmittels und der Base in dieser Reihenfolge zu der Mischung, um eine Reaktionslösung zu erhalten, die eine Verbindung der Formel (II) umfasst;
das Konzentrieren der Reaktionslösung; und
die Zugabe von Wasser zu der konzentrierten Reaktionslösung, um eine Verbindung der Formel (II) auszufällen.

12. Verfahren zur Herstellung von 5-Amino-3 substituiertem 1,2,4-Thiadiazol gemäss irgendeinem der Ansprüche 1 bis 11, wobei die Menge an zu der Mischung zugegebenem Bromierungsmittel das 0,5- bis 5,0-fache auf Molbasis, bezogen auf die Gesamtmenge der Amidinverbindung der Formel (I) und des Mineralsäuresalzes davon, beträgt.

13. Verfahren zur Herstellung von 5-Amino-3 substituiertem 1,2,4-Thiadiazol gemäss irgendeinem der Ansprüche 1 bis 12, wobei die Menge an zu der Mischung zugegebener Base das 0,5- bis 6,0-fache auf Molbasis, bezogen auf die Gesamtmenge der Amidinverbindung der Formel (I) und des Mineralsäuresalzes davon, beträgt.

14. Verfahren zur Herstellung von 5-Amino-3 substituiertem 1,2,4-Thiadiazol gemäss irgendeinem der Ansprüche 1 bis 13, wobei der Gehalt an Alkalimetallsalz der Thiocyansäure in der Mischung das 0,7- bis 5,0-fache auf Molbasis, bezogen auf den Gesamtgehalt der Amidinverbindung der Formel (I) und des Mineralsäuresalzes davon, beträgt.

15. Verfahren zur Herstellung von 5-Amino-3 substituiertem 1,2,4-Thiadiazol gemäss irgendeinem der Ansprüche 1 bis 14, wobei der Gesamtgehalt der Amidinverbindung der Formel (I), des Mineralsäuresalzes davon und des Alkalimetallsalzes der Thiocyansäure in der Mischung 1 bis 40 Masse-%, bezogen auf die Gesamtmenge der Mischung, beträgt.

## Revendications

1. Procédé de fabrication de 5-amino-3 substitué-1,2,4-thiadiazole, comprenant :
l'obtention d'un composé représenté par la Formule (II) suivante en ajoutant un agent de bromation et une base dans cet ordre à un mélange comprenant un alcool, au moins un sélectionné dans le groupe constitué d'un composé amidine représenté par la Formule (I) suivante et d'un sel d'acide minéral de celui-ci, et un sel de métal alcalin d'acide thiocyanique : 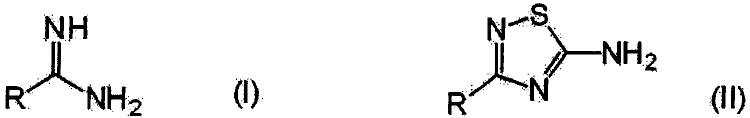
dans lequel, dans les Formules (I) et (II), R représente un groupe alkyle ayant 1 à 5 atomes de carbone ou un groupe alkylthio ayant 1 à 5 atomes de carbone.

2. Procédé de fabrication de 5-amino-3 substitué-1,2,4-thiadiazole selon la revendication 1, dans lequel le mélange a un pH de 9,0 à 11,5.

3. Procédé de fabrication de 5-amino-3 substitué-1,2,4-thiadiazole selon la revendication 1 ou 2, dans lequel l'alcool comprend un alcool primaire.

4. Procédé de fabrication de 5-amino-3 substitué-1,2,4-thiadiazole selon l'une quelconque des revendications 1 à 3, dans lequel l'agent de bromation comprend du brome.

5. Procédé de fabrication de 5-amino-3 substitué-1,2,4-thiadiazole selon l'une quelconque des revendications 1 à 4, dans lequel la base comprend une amine tertiaire.

6. Procédé de fabrication de 5-amino-3 substitué-1,2,4-thiadiazole selon l'une quelconque des revendications 1 à 5, dans lequel R dans les Formules (I) et (II) représente un groupe méthyle ou un groupe méthylthio.

7. Procédé de fabrication de 5-amino-3 substitué-1,2,4-thiadiazole selon l'une quelconque des revendications 1 à 6, dans lequel l'alcool comprend un alcool primaire ayant 1 à 3 atomes de carbone.

8. Procédé de fabrication de 5-amino-3 substitué-1,2,4-thiadiazole selon l'une quelconque des revendications 1 à 7, dans lequel la base comprend une amine tertiaire ayant 3 à 9 atomes de carbone.

9. Procédé de fabrication de 5-amino-3 substitué-1,2,4-thiadiazole selon l'une quelconque des revendications 1 à 8, dans lequel l'alcool comprend du méthanol.

10. Procédé de fabrication de 5-amino-3 substitué-1,2,4-thiadiazole selon l'une quelconque des revendications 1 à 9, dans lequel la base comprend de la triéthylamine.

11. Procédé de fabrication de 5-amino-3 substitué-1,2,4-thiadiazole selon l'une quelconque des revendications 1 à 10, dans lequel l'obtention du composé représenté par la Formule (II) comprend :
ajouter l'agent de bromation et la base dans cet ordre au mélange pour obtenir une solution réactionnelle comprenant le composé représenté par la Formule (II) ; concentrer la solution réactionnelle ; et
ajouter de l'eau à la solution réactionnelle concentrée pour précipiter le composé représenté par la Formule (II).

12. Procédé de fabrication de 5-amino-3 substitué-1,2,4-thiadiazole selon l'une quelconque des revendications 1 à 11, dans lequel une quantité de l'agent de bromation ajouté au mélange est de 0,5 fois à 5,0 fois en termes de mole par rapport à une quantité totale du composé amidine représenté par la Formule (I) et du sel d'acide minéral de celui-ci.

13. Procédé de fabrication de 5-amino-3 substitué-1,2,4-thiadiazole selon l'une quelconque des revendications 1 à 12, dans lequel une quantité de la base ajoutée au mélange est de 0,5 fois à 6,0 fois en termes de mole par rapport à une quantité totale du composé amidine représenté par la Formule (I) et du sel d'acide minéral de celui-ci.

14. Procédé de fabrication de 5-amino-3 substitué-1,2,4-thiadiazole selon l'une quelconque des revendications 1 à 13, dans lequel dans le mélange, une teneur en sel de métal alcalin d'acide thiocyanique est de 0,7 fois à 5,0 fois en termes de mole par rapport à une teneur totale du composé amidine représenté par la Formule (I) et du sel d'acide minéral de celui-ci.

15. Procédé de fabrication de 5-amino-3 substitué-1,2,4-thiadiazole selon l'une quelconque des revendications 1 à 14, dans lequel dans le mélange, une teneur totale en composé amidine représenté par la formule (I), en sel d'acide minéral de celui-ci, et en sel de métal alcalin d'acide thiocyanique est de 1 % en masse à 40 % en masse par rapport à une quantité totale du mélange.
